# FASCICULE DE BREVET EUROPEEN

(11) **EP 3 481 486 B1**
(45) Date de publication et mention de la délivrance du brevet: **02.09.2020**
(21) Numéro de dépôt: 17735162.4
(22) Date de dépôt: 07.07.2017
(51) Int. Cl.: A61M 27/00, A61B 5/03, A61B 5/00

(54) **DISPOSITIF DE DÉTECTION D'UN DYSFONCTIONNEMENT DE DÉRIVATION DE TYPE VENTRICULO-PÉRITONÉALE POUR LIQUIDE CÉPHALO-RACHIDIEN**
VORRICHTUNG ZUR ERKENNUNG EINER FEHLFUNKTION EINES VENTRIKULO-PERITONEALEN SHUNTS FÜR LIQUOR
DEVICE FOR DETECTING A MALFUNCTIONING OF A VENTRICULOPERITONEAL SHUNT FOR CEREBROSPINAL FLUID

(30) Priorité: 07.07.2016 FR 1656564
(43) Date de publication de la demande: 15.05.2019
(73) Titulaire: NEURALLYS, 75008 Paris (FR)
(72) Inventeur: AUVRAY, Philippe, 14000 CAEN (FR); MIREAU, Etienne, 95110 SANNOIS (FR)
(74) Mandataire: Regimbeau
(86) Numéro de dépôt international: PCT/EP2017/067058
(87) Numéro de publication internationale: WO 2018/007574

(56) Documents cités:
- WO-A1-2015/109260
- US-A1- 2007 208 293
- US-A1- 2008 139 959
- US-A1- 2014 243 703
- US-A1- 2014 276 346

## Description

### DOMAINE TECHNIQUE GÉNÉRAL ET ART ANTÉRIEUR

La présente invention est relative au domaine des valves de dérivation et plus particulièrement à celles utilisées dans les cas de traitement de l'hydrocéphalie.

Elle trouve avantageusement application dans le cas de valves ventriculo-péritonéales.

L'hydrocéphalie est une pathologie qui touche un nombre de patients important et qui peut avoir différentes causes : congénitale, congénitale en relation au virus Zika, tumeur cérébrale, suite de traumatisme crânien grave (chute, accident), dérèglement du système de régulation du liquide céphalo-rachidien (LCR), etc.

Cette pathologie engendre différents types de troubles : céphalées, nausées, troubles de la conscience, troubles visuels, comas voire décès dus à l'hypertension intracrânienne engendrée.

Le principal traitement connu consiste à implanter sur le patient une dérivation ventriculo-péritonéale composée de deux cathéters et d'une valve qui régule la pression intracrânienne et permet d'écouler le LCR qui est produit de manière continue dans le cerveau à raison de 21ml/h.

Ces valves connaissent toutefois des dérèglements et/ou dysfonctionnements parfois liés à des infections ou changements de la composition biochimique du LCR et doivent donc être souvent remplacées : 40% des implants sont remplacés après deux ans, 98% le sont après dix ans.

En outre, les diagnostics sur les dysfonctionnements de valves sont souvent difficiles et longs, nécessitent des chirurgies exploratoires parfois inutiles pour vérifier le bon fonctionnement de l'ensemble valve cathéters, ce qui n'est pas satisfaisant compte tenu des conséquences graves que ces dérèglements peuvent avoir sur la santé et les conditions de vie des patients. De plus, lorsque l'état de santé du patient ne s'améliore pas après plusieurs interventions chirurgicales, les médecins ont alors recours à des sondes intra-parenchymateuses (très invasives dans le cerveau) et nécessitant une hospitalisation, pour mesurer la pression intracrânienne et promulguer un diagnostic.

On connait déjà, par la demande US2008/0139959, un dispositif de mesure de pression intracrânienne implantable.

Le liquide céphalo-rachidien circule dans une chambre, qui est séparée, par une membrane souple, d'une chambre de mesure où se trouve le capteur de pression.

D'autres systèmes de shunt de dérivation à mesure de pression sont également connus par US 2007/0208293 et US 2014/0243703.

On connait en outre par la demande WO2015/109260 un dispositif de détection de détection d'un dysfonctionnement d'un ensemble valve cathéters de dérivation d'un liquide céphalo-rachidien selon le préambule de la revendication 1.

### PRÉSENTATION GÉNÉRALE DE L'INVENTION

L'invention propose une solution permettant de détecter un dysfonctionnement d'une valve de dérivation ventriculo-péritonéale et des cathéters qui lui sont associés, par le biais d'une mesure directe et ambulatoire de la pression intracrânienne, mesure déclenchée soit le patient lui-même, soit par un praticien.

Le diagnostic d'un dysfonctionnement d'un « shunt » ne peut se faire par le fait d'une mesure ponctuelle mais par de multiples acquisitions sur une période de temps allant de quelques jours à plusieurs semaines.

Notamment, un but général de l'invention est de proposer un dispositif permettant une meilleure détection et prévention des dysfonctionnements éventuels d'une valve intracrânienne et des cathéters associés.

Un autre but de l'invention est de proposer une solution minimisant les risques d'éventuels dépôts (débris cellulaires ou protéiques contenus dans le LCR) au niveau de la chambre dont la pression est mesurée et dans laquelle le liquide céphalo-rachidien circule.

Un autre but encore de l'invention est de proposer un dispositif de détection de dysfonctionnement permettant une parfaite isolation du capteur de pression et de la partie électronique par rapport au circuit de circulation du liquide céphalo-rachidien.

Un autre but également est de proposer une solution permettant d'intervenir sur l'électronique du dispositif, notamment pour la remplacer, sans avoir à interrompre et à ouvrir le circuit de circulation du liquide circulation du liquide céphalo-rachidien et à intervenir sur celui-ci.

Ainsi l'invention propose un dispositif de détection d'un dysfonctionnement d'un ensemble valve cathéters de dérivation d'un liquide céphalo-rachidien, selon la revendication 1. D'autres aspects sont présentés dans les revendications 2 à 13.

On notera qu'une telle structure permet la mesure de la pression dans la chambre dans laquelle circule le liquide céphalo-rachidien avec une meilleure sensibilité qu'avec l'art antérieur.

La membrane souple peut être un matériau à base de silicone et/ou de parylène.

La membrane et/ou les parois intérieures de la chambre peut être (ou peuvent être) recouverte(s) de parylène et/ou de PTFE.

La membrane peut recouvrir l'ensemble des parois de la chambre. La chambre peut présenter des formes générales arrondies, dépourvues d'angle.

On notera que les différentes caractéristiques ci-dessus contribuent à une mesure de pression sans perturbation de l'écoulement du liquide céphalo-rachidien et en limitant les risques de dépôt cellulaire ou protéique et donc d'obstruction dans la chambre.

Dans un mode de réalisation, la chambre est disposée à l'intérieur d'un boîtier en un matériau biocompatible, le canal laminaire étant adapté à un raccordement dudit boîtier à des cathéters de dérivation, en amont de la valve.

En variante, la chambre (comme le capteur et tout ou partie de l'électronique) peut être intégrée avec la valve dans une même enveloppe souple ou un même boîtier, pour former un seul ensemble.

Également, l'invention propose un ensemble comportant un tel dispositif et un lecteur très compacte et donc portable adapté pour communiquer avec l'électronique de contrôle et de communication du capteur.

De cette façon, on dispose d'une solution technique permettant une mesure ambulatoire et autonome de la pression du liquide dérivé en amont de la valve.

Un tel dispositif permet par exemple à une personne valvée de détecter par elle-même un dysfonctionnement de sa valve dont elle est implantée et de faire régulièrement des sondages et enregistrements qui pourront le cas échéant être ultérieurement soumis à des chirurgiens pour analyse.

Le dispositif pourrait être en outre complété par les différentes caractéristiques suivantes prises seules ou selon toutes leurs combinaisons techniquement possibles :
- il comporte une couche en titane interposée entre la membrane souple et le capteur ;
- un matériau inerte non compressible est interposé entre la membrane souple et le capteur ;
- un pion ou un gel est interposé entre la membrane souple et le capteur ;
- l'électronique de contrôle et de communication est montable/démontable indépendamment de la chambre et est adaptée pour un montage/démontage sans interruption de la circulation du liquide céphalo-rachidien ;
- la chambre est aménagée dans une coque s'étendant dans un boitier, ledit boitier étant adapté pour être monté sur un boitier recevant l'électronique de contrôle et de communication ;
- la coque est traversée par un trou ménagé dans ladite coque au droit de la membrane, ledit trou recevant le capteur de pression ;
- le capteur est monté sur une carte rigide qui s'étend d'une part à l'intérieur du boitier recevant la coque de la chambre et d'autre part à l'extérieur de celui-ci, ladite carte rigide présentant, sur sa partie à l'extérieur dudit boitier, des contacts pour la connexion électronique avec le boitier recevant boitier recevant l'électronique de contrôle et de communication ;
- les deux boîtiers comportent des picots et des orifices complémentaires de guidage/détrompage ;
- le capteur est un capteur piézo-électrique et/ou piézo-résistif ;
- l'électronique de contrôle et de communication comporte une électronique d'amplification, la mesure par le capteur étant une mesure passive ;
- l'électronique de contrôle comporte une électronique de sollicitation en résonance, la mesure par le capteur étant une mesure dynamique ;
- l'électronique de contrôle et de communication comporte un système d'alimentation inductif ;
- l'électronique de contrôle et de communication comporte une pile ou une batterie rechargeable ;
- le dispositif comporte en outre au moins un autre capteur pour l'analyse du liquide circulant dans le canal de circulation ;
- le boîtier est en titane ou en céramique ;
- le boîtier comporte une fenêtre en céramique pour permettre le passage des ondes électromagnétiques.

### PRÉSENTATION DES FIGURES

D'autres caractéristiques et avantages de l'invention ressortiront encore de la description qui suit, laquelle est purement illustrative et non limitative, et doit être lue en regard des figures annexées sur lesquelles :
- la figure 1 illustre un enfant sur lequel une dérivation ventriculo-péritonéale a été posée ;
- la figure 2 représente schématiquement une valve de dérivation et en amont de celle-ci un dispositif implantable conforme à un mode de réalisation possible pour l'invention ;
- la figure 3 illustre schématiquement l'interaction entre un tel dispositif implantable et un lecteur externe ;
- la figure 4 représente schématiquement en vue en perspective un dispositif implantable du type de celui illustré sur la figure 2, ce dispositif étant en l'occurrence d'un type réalisé par impression 3D ;
- les figures 5a et 5b d'une part et 6a et 6b d'autre part illustrent deux autres modes de réalisation possibles pour un tel dispositif implantable ;
- les figures 7a et 7b illustrent deux exemples de configurations d'implantations possibles ;
- les figures 8a et 8b illustrent schématiquement deux types d'électroniques de communication envisageables ;
- la figure 9 illustre un autre exemple de chambre de mesure ;
- la figure 10 illustre un mode de mise en place possible du capteur sur la chambre et sa membrane ;
- les figures 11a et 11b illustrent un autre mode de réalisation possible du dispositif de l'invention ;
- les figures 12a et 12b illustrent la connectique mécanique et électronique de cet autre mode de réalisation.

### DESCRIPTION D'UN OU PLUSIEURS MODES DE MISE EN ŒUVRE ET DE RÉALISATION

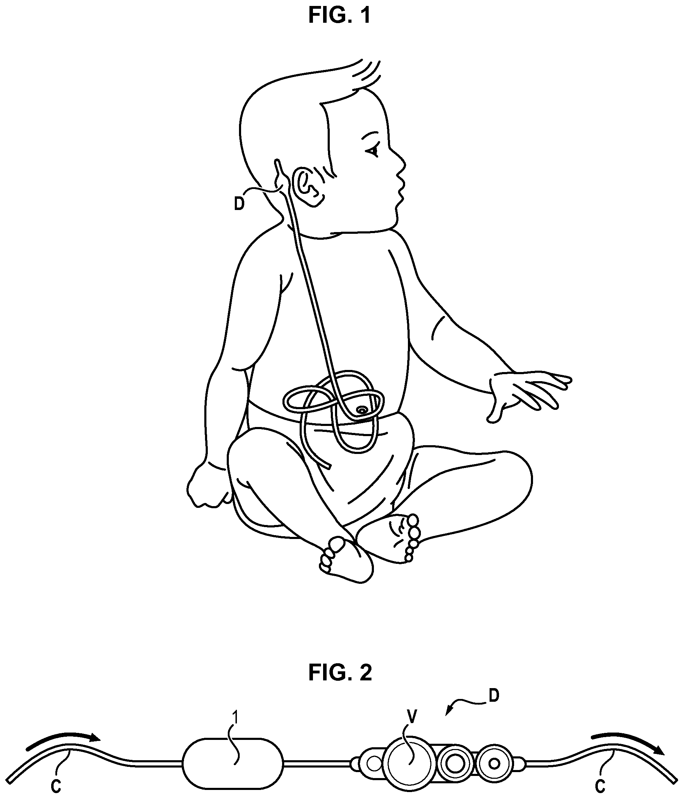

On a représenté sur la figure 1 un enfant sur lequel une dérivation D ventriculo-péritonéale a été posée.

Ainsi que l'illustre la figure 2, cette dérivation D est généralement constituée de cathéters C en amont et en aval d'une valve V qui s'ouvre pour assurer l'écoulement du liquide céphalo-rachidien en excès lorsque la pression intracrânienne augmente.

Sur cette même figure 2, on a en outre représenté un dispositif 1 formant capteur de pression qui est implanté en amont de la dérivation D.

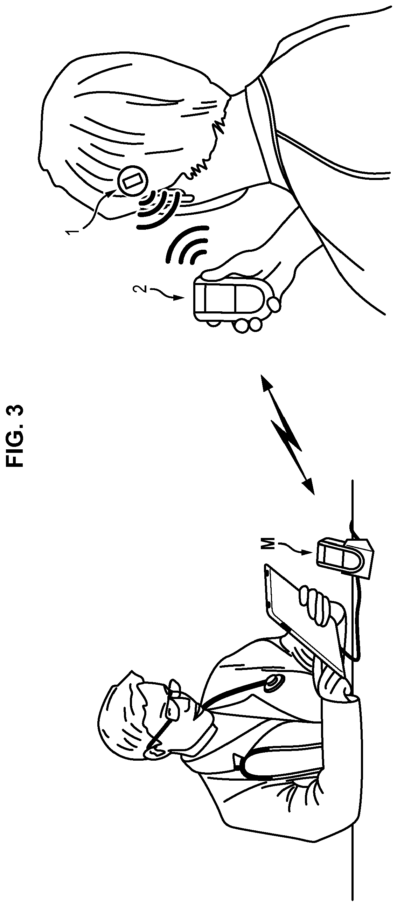

Ainsi que l'illustre la figure 3, ce dispositif 1 est destiné à communiquer avec un lecteur 2 externe.

Ce lecteur 2 intègre lui-même des moyens de communication lui permettant d'échanger avec des moyens M à distance chez un médecin (lecteur similaire au lecteur 2, lui-même relié à une tablette ou plus généralement à des moyens informatiques (PC, serveur d'un réseau, etc...) permettant de traiter et mémoriser les différentes informations lues par les lecteurs 2 et transmises aux moyens M). Ce lecteur 2 utilisé par le patient peut aussi être ramené par ce dernier auprès du praticien pour pouvoir décharger toutes les données enregistrées par le patient, par des liaisons informatiques (USB, Bluetooth...) et ainsi poser un diagnostic.

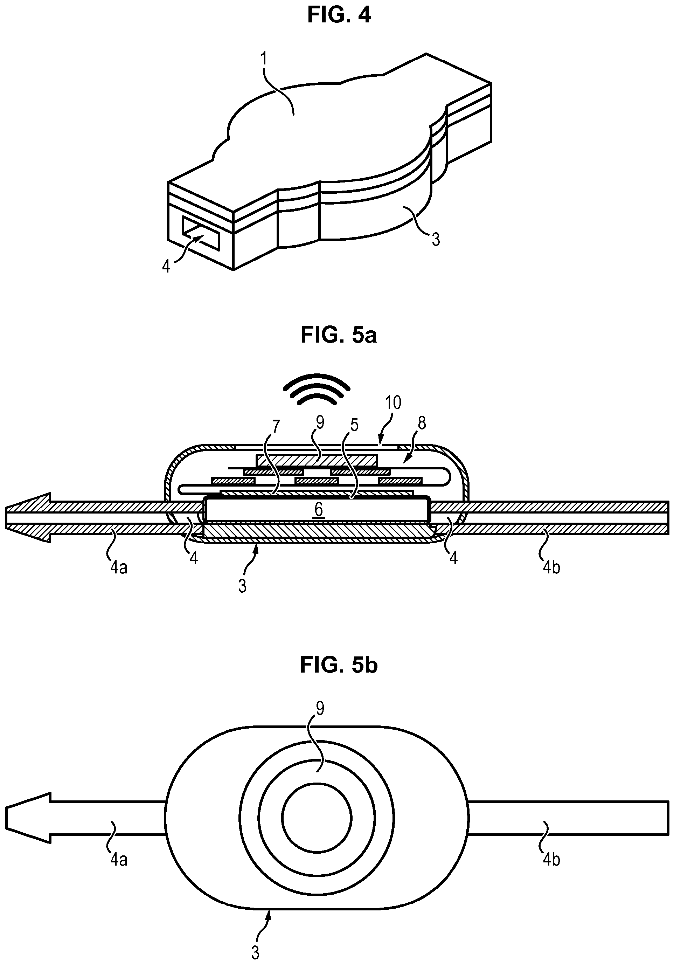

Le dispositif 1 se présente sous une forme rigide ou semi-rigide (figure 4) ou encore sous la forme d'une enveloppe souple. Il peut être séparé de la valve V ou au contraire être intégré avec la valve dans une même enveloppe souple ou un même boîtier, pour former un seul ensemble.

Il est de dimensions similaires à celles de la dérivation D, soit environ 47 x 17 x 10 mm³ (indications données à titre d'ordre de grandeur).

Comme l'illustrent les figures 4, 5a et 5b, ainsi que 6a et 6b, différentes configurations sont possibles pour ledit capteur 1.

Dans les exemples illustrés sur ces figures, il se présente par exemple sous la forme d'une structure qui comporte :
- un boîtier semi-rigide ou rigide 3,
- un canal 4 qui traverse le boîtier 3 et qui permet une circulation laminaire du liquide céphalo-rachidien, évitant une stagnation du LCR pouvant causer des infections et des obstructions,
- une membrane souple de séparation 5 qui ferme le canal 4 et est par exemple dans le même matériau que le boîtier 3,
- une chambre de mesure 6,
- un capteur 7 piézo-électrique ou piézo-résistif disposé dans ladite chambre 6 et rapporté sur la membrane 5,
- un circuit 8 avec différents composants électroniques,
- une antenne 9 qui est intégrée audit circuit ou est reliée à celui-ci.

La membrane 5 et le boîtier 3 sont en un ou des matériaux biocompatibles.

Par exemple, ledit boîtier 3 et la membrane 5 peuvent être en des matériaux polymères hydrophobes à base de silicone et/ ou de parylène éventuellement recouvert d'un matériau à base de polytétrafluoroéthylène (PTFE ou Téflon®).

La membrane 5 - et plus généralement - l'ensemble des parois intérieures de la chambre peuvent être recouverts de parylène afin de permettre une parfaite étanchéité et d'éviter tout accrochage de particule lors de l'écoulement du liquide CR dans la chambre 6.

Dans le cas où le boitier 3 est rigide, celui-ci peut être en des métaux biocompatibles (titane) ou des céramiques.

Par exemple, le boîtier peut être principalement en titane avec au moins une fenêtre 10 en une céramique transparente aux ondes électromagnétiques pour permettre la communication avec l'extérieur.

Le canal de circulation 4 qui traverse ledit boîtier 3 est adapté à un raccordement à une dérivation dudit liquide en amont de la valve. Notamment, il débouche dans la chambre de mesure 6, d'un côté et de l'autre de celle-ci. Il assure la circulation du liquide dérivé à travers ladite chambre 6 et ledit boîtier 3.

Il est par exemple constitué par un embout amont 4a et un embout aval 4b qui débouchent chacun dans la chambre 6 et permettent, dans leur partie extérieure au boîtier, un raccordement à des cathéters du circuit de dérivation. Les embouts 4a et 4b sont d'un type permettant de se raccorder à n'importe quel type de valve (réglable, à débit constant et à pression constante), quel que soit le fournisseur.

L'embout 4b est par exemple directement raccordé à la valve, ce qui permet d'éviter de remettre un bout de cathéter entre le dispositif et la valve et évite d'introduire une perturbation dans la circulation du LCR.

La membrane 5 qui porte le capteur 7 peut être isolée de celui-ci par une couche formant barrière isolante en titane.

La chambre de mesure 6 a des formes générales arrondies, sans angle, afin de limiter les perturbations d'écoulements pour le liquide LCR.

La chambre 6 a par exemple une forme circulaire plate ou encore une forme générale en ellipsoïde allongé.

Le canal 4 débouche dans ladite chambre 6 au niveau de deux extrémités diamétralement opposées.

Le capteur 7 est un capteur à membrane de Si, qui est par exemple du type de celui décrit dans la demande de brevet WO2014187937, d'autres types de capteurs étant néanmoins possibles.

Il est de forme et de dimensions complémentaires à celles de la chambre de mesure 6 et est par exemple constitué par un disque de 1 cm de diamètre et de 50 µm d'épaisseur portant une ou plusieurs électrodes au centre ou en périphérie.

Le circuit 8 peut être un circuit imprimé flexible.

Il peut porter l'antenne 9, qui est par exemple alors une antenne bobinée ainsi que cela sera décrit plus loin de façon plus détaillée (figures 5a et 5b).

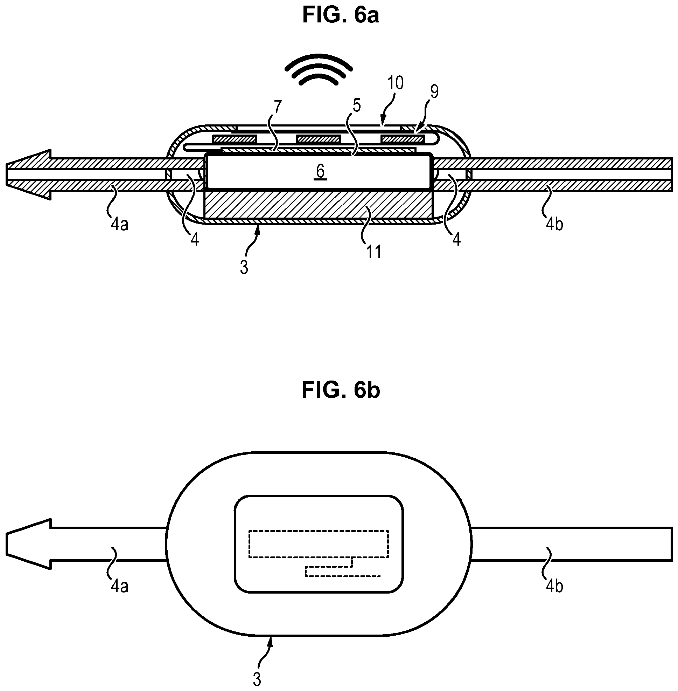

En variante, l'antenne 9 peut être portée par un circuit PCB simple face collé sur le boîtier 3, sur une face intérieure de celui-ci (figures 6a et 6b). Différentes architectures sont possibles pour le capteur piézo-électrique 7 à l'intérieur du dispositif 1.

Dans une première variante, le capteur piézo-électrique peut fonctionner en mode passif (naissance de la charge sous l'effet d'une déformation) en étant associé à un amplificateur au niveau de l'électronique 8.

La pression mesurée par le capteur 7 et relevée par le lecteur 2 est alors analysée pour en déduire le cas échéant l'existence d'une surpression dans le canal de circulation 4. En particulier, plus la pression augmente dans celui-ci, plus la membrane souple 5 - qui se déforme - devient rigide. Le signal recueilli (qui correspond aux pulsations cardiaques) est atténué.

L'analyse de ce signal peut être faite au niveau du lecteur 2 ou à distance, par les moyens informatiques associés au lecteur M avec lequel ledit lecteur 2 communique. Le niveau d'atténuation peut être comparé à un seuil préalablement déterminé pour chaque patient. La détection du franchissement de ce seuil peut par exemple déclencher l'émission d'un signal d'alerte à destination de l'utilisateur du lecteur.

Dans une autre variante, le capteur piézo-électrique 7 peut être sollicité en résonnance par l'électronique 8 dans un mode dynamique.

Le lecteur 2 ou l'appareil à distance analyse alors la variation de la fréquence de résonnance du capteur. L'existence d'une surpression dans le canal 4 va en effet entrainer un décalage de la fréquence de résonance de la membrane souple. La variation de la fréquence de résonance est par exemple là aussi comparée à un seuil, qui lorsqu'il est franchi déclenche l'émission d'un signal d'alerte.

Dans les modes nécessitant une excitation du capteur, l'électrode du capteur est centrale tandis que les électrodes de lecture sont sur le bord de la structure piézo-électrique.

Dans d'autres variantes encore, les électrodes d'excitation peuvent être situées du côté opposé au canal 4 de circulation du liquide.

Différentes implantations sont possibles pour le dispositif 1.

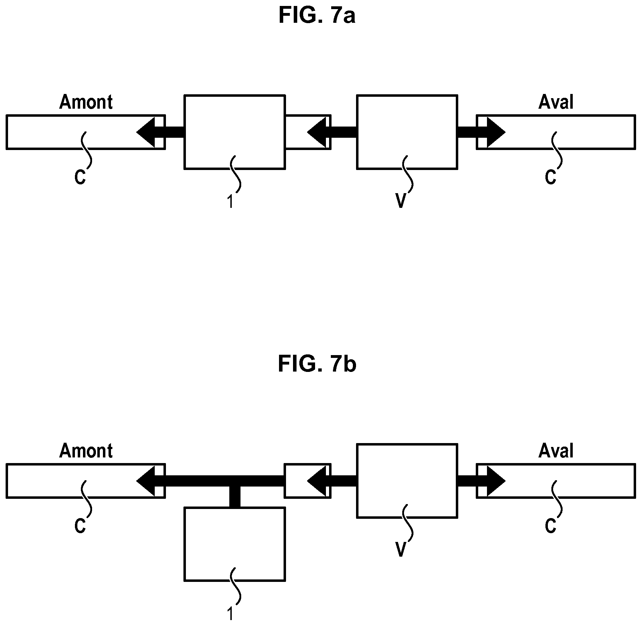

Notamment, ainsi qu'illustré sur la figure 7a, le dispositif 1 peut être disposé en série avec la valve V entre les deux cathéters amont et aval C.

Il peut également être disposé en dérivation (figure 7b).

Avec ces différentes implantations, le dispositif 1 est adapté pour mesurer, grâce au capteur piézo-électrique 7, une surpression en amont de la dérivation D.

Le déclenchement d'une mesure par le capteur 7 peut être commandé par interrogation par le lecteur 2. L'électronique 8 gère les échanges avec le lecteur 2 et contrôle l'actionnement de la mesure par le capteur piézo-électrique 7.

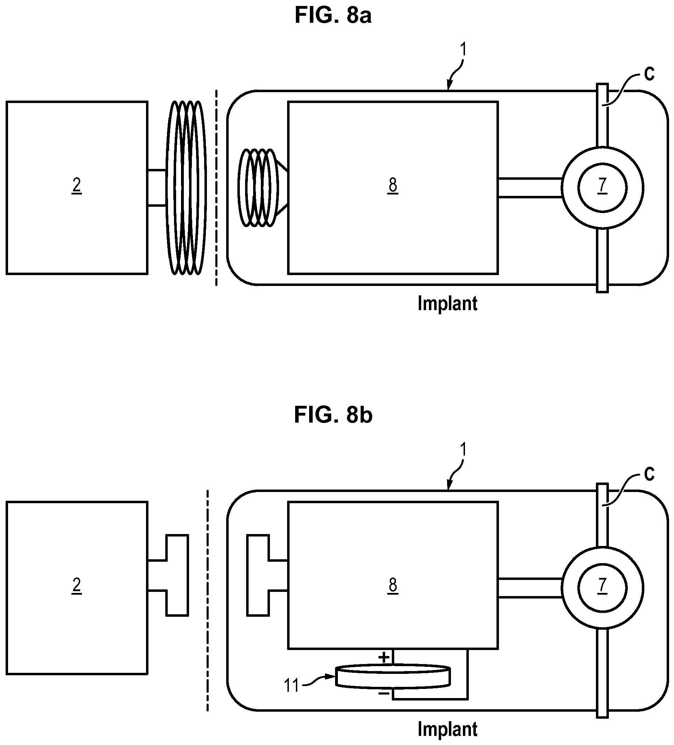

Ainsi que l'illustre la figure 8a, l'électronique 8 peut comprendre un système de télé-alimentation 9 qui permet de s'affranchir d'une pile embarquée et donne une longue durée de vie au dispositif.

Un tel système inductif sans pile fonctionne alors de préférence dans les bandes de fréquence 13,56 MHz, 27 MHz (bandes de fréquence ISM). L'antenne 9 est alors une antenne bobinée qui est par exemple disposée sur le circuit flexible 8 (mode de réalisation des figures 5a et 5b).

En variante (figure 6a et figure 8b), l'électronique 8 peut intégrer une pile 11 ou une batterie rechargeable, ce qui permet de plus grandes portées entre le lecteur 2 et le dispositif 1 implanté, celui-ci intégrant une électronique de communication radio fréquence.

Cette variante permet en outre de programmer des mesures répétitives sans intervention par prise de pression semi-continues.

Dans ce mode de réalisation, le lecteur 2 réveille l'implant (par la biais d'un signal radio fréquence, comportant une signature numérique d'appairage et émis dans la bande de fréquence 2.4GHz) que constitue le capteur 1 en demandant une mesure, puis ledit implant se remet en mode veille après avoir transmis le résultat de la mesure, afin d'économiser la batterie.

Un tel dispositif avec pile fonctionne dans les bandes de fréquence réservées 402 MHz (bande MICS) ou 433 MHz (bande ISM).

En variante encore, le dispositif 1 peut intégrer d'autres capteurs que des capteurs de mesure de type piézo-électrique : capteurs aptes à mesurer le pH ou à faire une analyse biochimique du LCR pour détecter une infection, etc.

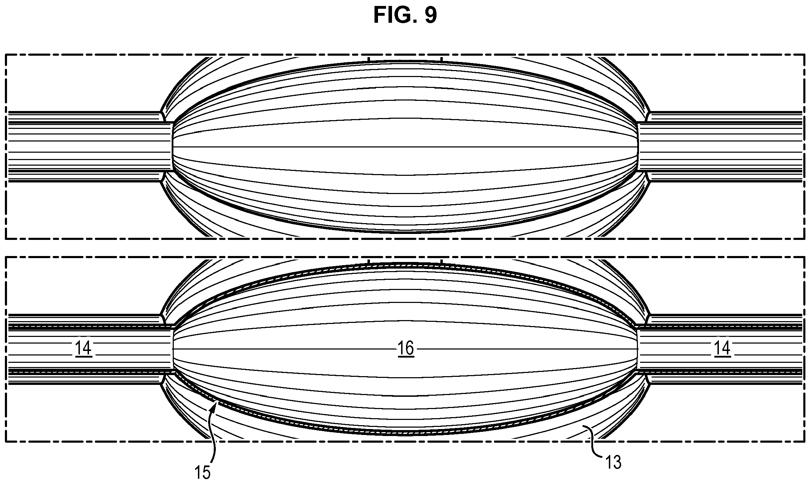

En référence à la figure 9, on a représenté une variante de chambre de mesure possible.

Dans cette variante, la chambre - référencée par 16 - à une forme générale en ellipsoïde allongée. Elle est aménagée et définie par une coque 13 indépendante du boitier où se trouve l'électronique.

Cette coque 13 est constituée par deux demi-coques rigides assemblées.

Chacune de ces demi-coques se prolonge par un lumen 14, les deux lumens 14 constituant les entrées et sorties du liquide LCR.

Ces demi-coques et les lumens d'entrée/sortie sont par exemple en Titane ou en une céramique.

Les dimensions de la chambre 16 sont par exemple les suivantes :
- diamètre du grand axe dans la direction de circulation du liquide LCR : 13 mm,
- diamètre du grand axe en son plan milieu : 6 mm,
- diamètre du grand axe en son plan milieu : 5 mm.

Une membrane 15 souple est prévue qui recouvre l'ensemble de l'intérieur de la chambre 16 ainsi que l'intérieur des deux lumens 14 d'entrée et de sortie.

Cette membrane 15 est par exemple en un matériau polymère à base de silicone et/ou parylène avec possibilité de recouvrir avec un matériau à base de polytétrafluoroéthylène (PTFE ou Téflon®) ou de parylène.

Son épaisseur est comprise entre 1000 nm et 100 nm pour du parylène et comprise entre 100um et 900um pour du silicone.

Une telle disposition de membrane, dans laquelle ladite membrane recouvre l'ensemble de l'intérieur de la chambre 16, permet d'assurer une parfaite étanchéité et séparation entre la circulation du liquide LCR et l'électronique du dispositif.

En outre, elle contribue à éviter toute aspérité pour le liquide LCR circulant dans la chambre 16, évitant ainsi les risques de dépôt et de bouchage.

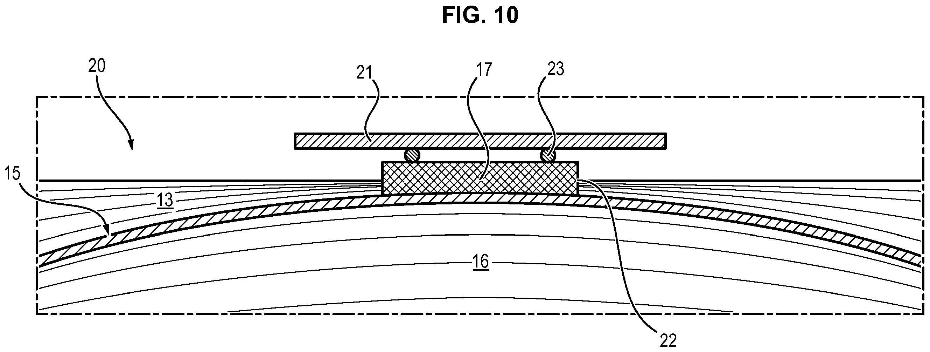

Ainsi que l'illustre la figure 10 et les figures suivantes, la coque 13 est montée à l'intérieur d'un boitier 20, lui-même indépendant du boitier où se trouve l'électronique.

Ce boitier 20 intègre un circuit imprimé rigide ou semi rigide 21, qui est fixé à l'intérieur dudit boîtier 20 et qui porte un capteur de pression 17, par exemple de type piézo-électrique ou piézo-résistif.

Un trou 22 est à cet effet ménagé dans la coque 13. Ce trou 22 traverse l'épaisseur dudit boitier pour permettre au capteur 17 d'être en contact avec la membrane 15. Le cas échéant, le capteur 17 comporte lui-même une membrane de mesure et le transfert de pression entre cette membrane et la membrane 15 se fait à travers un pion intermédiaire ou à travers un gel ou plus généralement tout inerte matériau non compressible.

Le capteur 17 et le trou 22 sont par exemple de dimensions 1x1 mm.

On notera que le montage ainsi réalisé permet une parfaite séparation entre d'un côté l'électronique et de l'autre le liquide LCR circulant, tout en permettant un montage du capteur sur la membrane, ce qui autorise une plus grande précision de mesure.

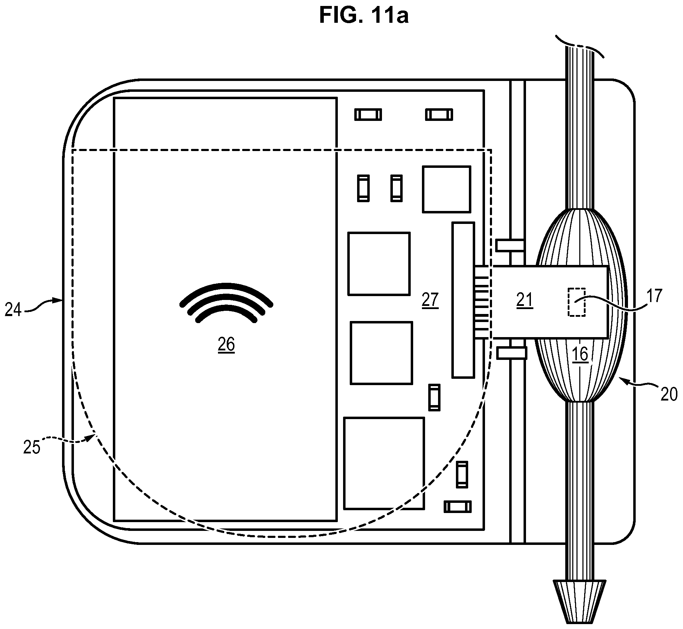

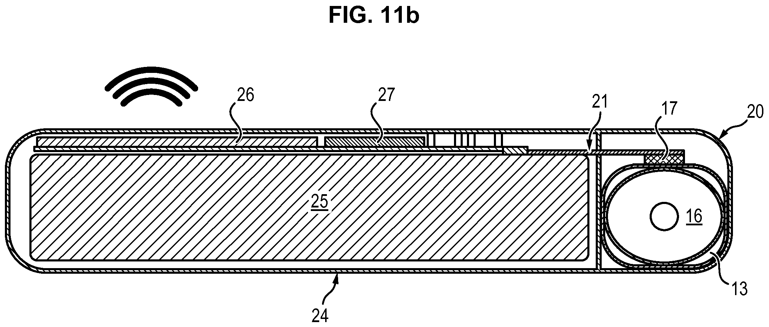

Le montage du boitier 20 sur le boitier d'électronique (référencé par 24) est plus particulièrement illustré sur les figures 11a, 11b.

Le boitier d'électronique 24 intègre une batterie 25, une antenne planaire 26 et un circuit souple 27 sur lequel sont montés les différents composants électroniques.

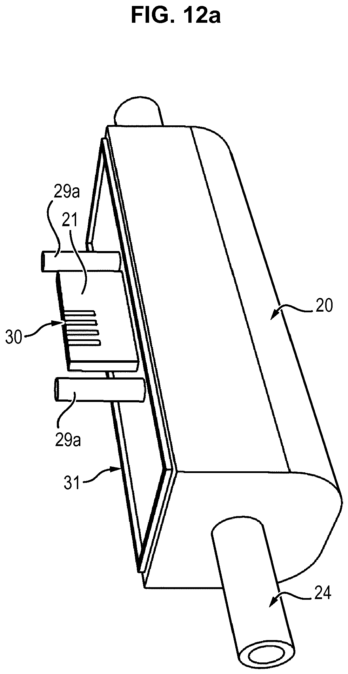

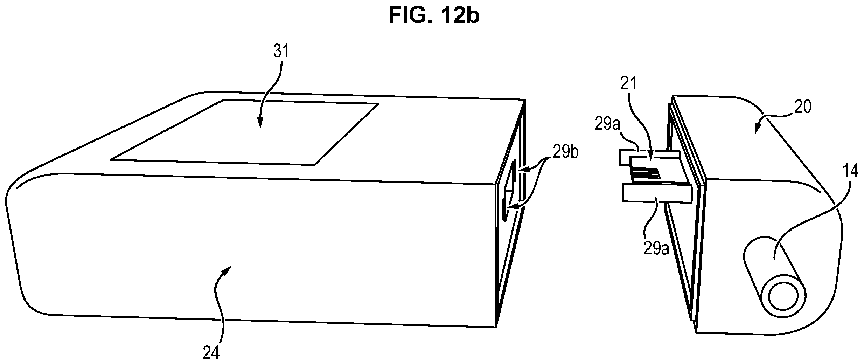

Le boitier 20 se connecte mécaniquement et électroniquement au boîtier 24 de la façon qui est illustrée sur les figures 12a, 12b.

Comme plus particulièrement visible sur ces figures, le circuit imprimé rigide 21 s'étend à l'extérieur du boitier 20 et est destiné à s'engager dans une fente de guidage complémentaire 28 que présente le boitier 24.

Une fermeture 31 permet le montage initial de la coque 13, du capteur 17 et du circuit 21 dans le boitier 20. Cette fermeture 21 assure l'étanchéité du boitier 20 une fois l'ensemble mis en place.

Des picots 29a et des orifices complémentaires 29b sont également ménagés sur le boitier 24 ou le boitier 20 pour assurer le guidage et le détrompage lors de la mise en place du boitier 24 sur le boitier 20.

Des contacts 30 sont prévus au niveau du bord de la carte 21 qui viennent coopérer avec des contacts complémentaires du circuit souple.

Ainsi, le boitier 24 d'électronique se connecte et se déconnecte aisément par rapport au boitier 20. Il peut donc être changé sans nécessiter d'intervention et de modification au niveau de l'implant lui-même, ce dernier restant branché dans le circuit de circulation du liquide céphalo-rachidien tandis que la partie électronique et la batterie sont changés. Le débranchement des cathéters de circulation de LCR n'ayant pas lieu, le risque d'infection lié à ce débranchement est donc écarté.

Sur la figure 12b, on a également représenté une fenêtre 31 en céramique ménagée sur le boitier 24 pour l'émission/réception de l'antenne RF (boitier 24 en titane).

## Revendications

1. Dispositif de détection d'un dysfonctionnement d'un ensemble valve cathéters de dérivation d'un liquide céphalo-rachidien, comportant :
• une chambre (6, 16) ;
• un canal laminaire (4, 14) qui débouche dans ladite chambre (6, 16), d'un côté et de l'autre de celle-ci, et qui est adapté pour assurer la circulation d'un liquide dérivé, à travers ladite chambre (6, 16) ;
• un capteur (7, 17) de mesure de pression pour la mesure d'une pression dans ladite chambre (6, 16) ;
• une membrane souple (5, 15) qui sépare le capteur (7, 17) de la chambre (6, 16) ;
• une électronique (8, 9) de contrôle et de communication apte à échanger avec un lecteur externe pour transmettre une mesure ambulatoire par ledit capteur (7, 17),
le capteur (7, 17) étant rapporté sur la membrane souple (5, 15) avec laquelle il est en contact,
**caractérisé en ce que** les parois de la chambre sont définies par un boitier rigide ou une coque, la membrane (5, 15) recouvrant l'ensemble des parois de ladite chambre (6, 16), le dispositif étant apte à être implanté en amont d'une valve intracraniennede l'ensemble de dérivation pour la mesure directe de la pression intracrânienne par le capteur de pression.

2. Dispositif selon la revendication 1, dans lequel la chambre (6, 16) est définie par une coque (13) de forme générale en ellipsoïde allongée, ladite coque (13) étant traversée par un trou (22) permettant la mise en contact du capteur (7, 17) avec la membrane (5, 15).

3. Dispositif selon la revendication 1, **caractérisé en ce que** la membrane souple est un matériau à base de silicone et/ou parylène.

4. Dispositif selon la revendication 1, **caractérisé en ce que** la membrane est recouverte de parylène et/ou de PTFE.

5. Dispositif selon la revendication 1, **caractérisé en ce qu'**il comporte une couche en titane interposée entre la membrane souple et le capteur.

6. Dispositif selon la revendication 1, **caractérisé en ce qu'**un matériau inerte non compressible est interposé entre la membrane souple et le capteur.

7. Dispositif selon la revendication 6, **caractérisé en ce qu'**un pion ou un gel est interposé entre la membrane souple et le capteur.

8. Dispositif selon la revendication 1, **caractérisé en ce que** l'électronique de contrôle et de communication est montable/démontable indépendamment de la chambre et est adaptée pour un montage/démontage sans interruption de la circulation du liquide céphalo-rachidien.

9. Dispositif selon la revendication 8, **caractérisé en ce que** la coque s'étend dans un boitier, ledit boitier étant adapté pour être monté sur un boitier recevant l'électronique de contrôle et de communication.

10. Dispositif selon la revendication 9, **caractérisé en ce que** le capteur est monté sur une carte rigide qui s'étend d'une part à l'intérieur du boitier recevant la coque de la chambre et d'autre part à l'extérieur de celui-ci, ladite carte rigide présentant, sur sa partie à l'extérieur dudit boitier, des contacts pour la connexion électronique avec le boitier recevant l'électronique de contrôle et de communication.

11. Dispositif selon l'une des revendications 9 ou 10, **caractérisé en ce que** les deux boîtiers comportent des picots et des orifices complémentaires de guidage/détrompage.

12. Dispositif selon l'une des revendications 9 à 11, **caractérisé en ce que** le boîtier comporte une fenêtre en céramique pour permettre le passage des ondes électromagnétiques.

13. Ensemble comportant un dispositif selon l'une des revendications précédentes et un lecteur adapté pour communiquer avec l'électronique de contrôle et de communication du capteur.

## Patentansprüche

1. Vorrichtung zur Erkennung einer Fehlfunktion einer Ableitungsventileinheit für Liquor, Folgendes beinhaltend:
• eine Kammer (6, 16);
• einen Laminarkanal (4, 14), der auf der einen Seite und der anderen derselben in die Kammer (6, 16) mündet, und der angepasst ist, um für eine Zirkulation einer abgeleiteten Flüssigkeit durch die Kammer (6, 16) hindurch zu sorgen;
• einen Druckmesssensor (7, 17) zum Messen eines Drucks in der Kammer (6, 16);
• eine flexible Membran (5, 15), die den Sensor (7, 17) von der Kammer (6, 16) trennt;
• eine Steuer- und Kommunikationselektronik (8, 9), die imstande ist, sich mit einem externen Lesegerät auszutauschen, um eine ambulante Messung durch den Sensor (7, 17) zu übertragen,
wobei der Sensor (7, 17) auf der flexiblen Membran (5, 15), mit der er in Kontakt steht, beigebracht ist,
**dadurch gekennzeichnet, dass** die Wände der Kammer durch ein starres Gehäuse oder eine Schale definiert sind, wobei die Membran (5, 15) die Gesamtheit der Wände der Kammer (6, 16) abdeckt, wobei die Vorrichtung in der Lage ist, stromaufwärts eines intrakraniellen Ventils der Ableiteinheit zum direkten Messen des intrakraniellen Drucks durch den Drucksensor eingepflanzt zu werden.

2. Vorrichtung nach Anspruch 1, wobei die Kammer (6, 16) durch eine Schale (13) in einer im Allgemeinen länglichen Ellipsenform definiert wird, wobei die Schale (13) durch ein Loch (22) durchquert wird, das die Kontaktaufnahme des Sensors (7, 17) mit der Membran (5, 15) ermöglicht.

3. Vorrichtung nach Anspruch 1, **dadurch gekennzeichnet, dass** die flexible Membran aus einem Material auf Basis von Silikon und/oder Parylene ist.

4. Vorrichtung nach Anspruch 1, **dadurch gekennzeichnet, dass** die Membran mit Parylene und/oder PTFE abgedeckt ist.

5. Vorrichtung nach Anspruch 1, **dadurch gekennzeichnet, dass** sie eine Schicht aus Titan beinhaltet, die zwischen der flexiblen Membran und dem Sensor eingesetzt ist.

6. Vorrichtung nach Anspruch 1, **dadurch gekennzeichnet, dass** ein nicht komprimierbares inertes Material zwischen der flexiblen Membran und dem Sensor eingesetzt ist.

7. Vorrichtung nach Anspruch 6, **dadurch gekennzeichnet, dass** ein Zapfen oder ein Gel zwischen der flexiblen Membran und dem Sensor eingesetzt ist.

8. Vorrichtung nach Anspruch 1, **dadurch gekennzeichnet, dass** die Steuer- und Kommunikationselektronik unabhängig von der Kammer montier-/demontierbar ist, und für eine Montage-Demontage ohne Unterbrechung der Zirkulation des Liquors angepasst ist.

9. Vorrichtung nach Anspruch 8, **dadurch gekennzeichnet, dass** sich die Schale in ein Gehäuse erstreckt, wobei das Gehäuse angepasst ist, um auf einem Gehäuse montiert zu werden, das die Steuer- und Kommunikationselektronik aufnimmt.

10. Vorrichtung nach Anspruch 9, **dadurch gekennzeichnet, dass** der Sensor auf einer starren Karte montiert ist, die sich einerseits im Inneren des Gehäuses, das die Schale der Kammer aufnimmt, und andererseits außerhalb desselben erstreckt, wobei die starre Karte auf ihrem Teil außerhalb des Gehäuses Kontakte für den elektronischen Anschluss an das Gehäuse aufweist, das die Steuer- und Kommunikationselektronik aufnimmt.

11. Vorrichtung nach einem der Ansprüche 9 oder 10, **dadurch gekennzeichnet, dass** die beiden Gehäuse Noppen und ergänzende Öffnungen zum Führen/ Schützen vor Verwechslung beinhalten.

12. Vorrichtung nach einem der Ansprüche 9 bis 11, **dadurch gekennzeichnet, dass** das Gehäuse ein Fenster aus Keramik beinhaltet, um den Durchgang der elektromagnetischen Wellen zu ermöglichen.

13. Einheit, eine Vorrichtung nach einem der vorstehenden Ansprüche und ein Lesegerät beinhaltend, das angepasst ist, um mit der Steuer- und Kommunikationselektronik des Sensors zu kommunizieren.

## Claims

1. Device for detecting a malfunction of a valve/catheter assembly for shunting a cerebrospinal fluid, including:
• a chamber (6, 16);
• a laminar channel (4, 14) which leads to said chamber (6, 16), on either side thereof, and which is suitable for ensuring the flow of a shunted fluid, via said chamber (6, 16);
• a pressure measurement sensor (7, 17) for measuring a pressure in said chamber (6, 16);
• a flexible membrane (5, 15) which separates the sensor (7, 17) from the chamber (6, 16);
• control and communication electronics (8, 9) suitable for exchanging with an external reader to transmit an ambulatory measurement by said sensor (7, 17),
the sensor (7, 17) being mounted on the flexible membrane (5, 15) with which it is in contact,
**characterised in that** the walls of the chamber are defined by a rigid box or a casing, the membrane (5, 15) covering all of the walls of said chamber (6, 16), the device being suitable for being implanted upstream of an intracranial valve of the shunting assembly for direct intracranial pressure measurement by the pressure sensor.

2. Device according to claim 1, wherein the chamber (6, 16) is defined by a casing (13) of general elongated ellipsoid shape, said casing (13) being traversed by a hole (22) enabling the sensor (7, 17) to be placed in contact with the membrane (5, 15).

3. Device according to claim 1, **characterised in** the flexible membrane is a material based on silicone and/or parylene.

4. Device according to claim 1, **characterised in that** the membrane is coated with parylene and/or PTFE.

5. Device according to claim 1, **characterised in that** it includes a titanium layer inserted between the flexible membrane and the sensor.

6. Device according to claim 1, **characterised in that** an incompressible inert material is inserted between the flexible membrane and the sensor.

7. Device according to claim 6, **characterised in that** a slug or a gel is inserted between the flexible membrane and the sensor.

8. Device according to claim 1, **characterised in that** the control and communication electronics are mountable/removable independently of the chamber and are suitable for mounting/removal without interrupting the flow of the cerebrospinal fluid.

9. Device according to claim 8, **characterised in that** the casing extends into a box, said box being suitable for being mounted onto a box receiving the control and communication electronics.

10. Device according to claim 9, **characterised in that** the sensor is mounted on a rigid board which extends, on one hand, inside the box receiving the casing of the chamber and, on the other, outside said box, said rigid board having, on the part thereof outside said box, contacts for the electronic connection with the box receiving the control and communication electronics.

11. Device according to one of claims 9 or 10, **characterised in that** the two boxes include barbs and complementary guiding/polarisation orifices.

12. Device according to one of claims 9 to 11, **characterised in that** the box includes a ceramic window to allow the passage of electromagnetic waves.

13. Assembly including a device according to one of the preceding claims and a reader suitable for communicating with the control and communication electronics of the sensor.
